Europäisches Patentamt

**European Patent Office**

Office européen des brevets

(11) Numéro de publication: **0 236 156**
A2

## DEMANDE DE BREVET EUROPEEN

(21) Numéro de dépôt: **87400095.3**

(22) Date de dépôt: **16.01.87**

(51) Int. Cl.⁴: **C 05 F 9/04**, C 05 F 11/08, C 12 N 11/02, C 12 N 1/04 // C12F3/06

(30) Priorité: **17.01.86 FR 8600626**
**17.01.86 FR 8600627**

(43) Date de publication de la demande: **09.09.87**
**Bulletin 87/37**

(84) Etats contractants désignés: **AT BE CH DE ES FR GB GR IT LI LU NL SE**

(71) Demandeur: **SOCIETE DE PRODUCTION ET COMMERCIALISATION DES ENGRAIS ET PHYTOS, 25, avenue de Bordeaux, F-11100 Narbonne (FR)**

(72) Inventeur: **Thierry, Alain, Residence La Patinoire Avenue de Vert Bois, F-34100 Montpellier (FR)**
Inventeur: **Chicheportiche, Robert, "Les Gours" Lot No 5§Route de Laverune, F-34100 Montpellier (FR)**

(74) Mandataire: **Warcoin, Jacques et al, Cabinet Régimbeau 26, avenue Kléber, F-75116 Paris (FR)**

(54) **Nouveau procédé de compostage, inoculum microbien comportant comme support, de la pulpe de raisin sechée et broyée et application, notamment, à l'enrichissement des sols.**

(57) La présente invention concerne un procédé de compostage de matières organiques d'origine végétale, caractérisé en ce que:
a) on prépare un tas de matières à composter contenant des matières organiques d'origine végétale et éventuellement des matières organiques d'origine animale,
b) on insuffle de l'oxygène dans le tas de matières à composter,
c) à l'apparition de la phase thermophile du compostage, on active la biodégradation par l'inoculation de bactéries sélectionnées et on procède à l'aération mécanique régulière du tas,
d) l'aération mécanique est arrêtée avant la fin de la phase thermophile.
La présente invention concerne en outre un inoculum microbien utile notamment à l'accélération du compostage à l'amélioration des sols et/ou à l'enrobage des graines, caractérisé en ce que le support microbien est constitué essentiellement par de la pulpe de raisin séchée et broyée.

0236156

## NOUVEAU PROCEDE DE COMPOSTAGE, INOCULUM MICROBIEN COMPORTANT COMME SUPPORT, DE LA PULPE DE RAISIN SECHEE ET BROYEE ET APPLICATION, NOTAMMENT, A L'ENRICHISSEMENT DES SOLS

La présente invention concerne un procédé de compostage ainsi qu'un inoculum microbien, utile à l'enrichissement des sols.

Comme cela est connu, le compostage est une fermentation aérobie de matières organiques d'origine végétale qui aboutit à l'obtention d'un produit organique humique stabilisé qui peut être utilisé pour enrichir les sols.

Suivant la nature des végétaux compostés, les caractéristiques des composts obtenus diffèrent.

En particulier, le procédé selon l'invention s'intéresse au compostage des déchets viticoles tels que pulpe sèche et pulpe humide, mais également rafle de raisin, ces déchets étant compostés avec du fumier de mouton.

Actuellement, la matière première de compostage dans ce domaine est la pulpe de raisin, résidu de distillation qui peut être obtenu sous deux formes : une forme sèche (10 % d'humidité) épépinée et broyée finement et une forme humide (60 à 65 % d'humidité) épépinée et broyée.

2

0236156

Le compostage s'effectue en andains et dure 3 mois avec la pulpe sèche, et plus de 6 mois avec la pulpe humide.

Le produit obtenu à partir de la pulpe sèche est de qualité supérieure à celui obtenu à partir de la pulpe humide. De ce fait, la pulpe sèche constitue le substrat par excellence qui est utilisé de façon préférentielle.

Cependant, la pulpe sèche est d'un prix de revient de deux à trois fois plus élevé que celui de la pulpe humide ou que la rafle. L'utilisation plus importante de ces deux derniers substrats pourraît abaisser d'une façon très importante le coût du produit fini à condition toutefois de réduire notablement leur temps de compostage.

La présente invention a plus particulièrement pour objet un procédé de compostage qui permet un gain de temps très important.

Il s'agit d'un procédé de compostage de matières organiques d'origine végétale, caractérisé en ce que :

a) on prépare un tas de matières à composter contenant des matières organiques d'origine végétale et éventuellement des matières organiques d'origine animale ;

b) on insuffle de l'oxygène dans le tas de matières à composter ;

c) à l'apparition de la phase thermophile du compostage, on procède à l'aération mécanique régulière du tas ;

d) l'aération mécanique est arrêtée avant la fin de la phase thermophile.

De façon préférentielle, dans l'étape b) l'oxygène est insufflé sans que le tas de compostage soit demembré, ceci pour permettre l'établissement de la flore microbienne.

Dans ces conditions, l'oxygène, sous forme d'air comprimé par exemple, est insufflé par l'intermédiaire de cannes perforées de façon que l'ensemble du volume du tas soit oxygéné.

Les essais qui ont été conduits systématiquement et qui sont reproduits aux exemples ont permis de mettre en évidence que ce type d'oxygénation était utile jusqu'au début de la phase thermophile. En fait, cette insufflation sera appliquée de préférence à partir du 2ème jour.

L'aération mécanique est mise en oeuvre de préférence durant quelques jours, environ 5 jours, au début de la phase thermophile.

Durant la phase d'oxygénation par insufflation d'oxygène, il convient de contrôler la température pour déterminer l'apparition de la phase thermophile mais également pour éviter les surchauffes locales ou temporaires. Il convient, en outre, de maintenir si possible l'humidité entre 45 et 55 % car le tas de compostage tend à se dessécher.

L'un des éléments "clé" de ce procédé est l'utilisation d'un inoculum microbien qui correspond au compostage effectué.

L'inoculum microbien utilisé est, de préférence, obtenu par isolement de microorganismes au niveau des différentes phases du compostage ; ces microorganismes étant sélectionnés par la technique des transferts successifs permettant d'obtenir les microorganismes dominants dégradants de chaque phase.

Ces microorganismes sont, en outre, choisis pour certaines de leurs propriétés dégradantes liées aux matières végétales à composter.

On sait, en effet, que les parois cellulaires des végétaux sont essentiellement constitué de cellulose, d'hémicellulose et de lignine. Il s'agit de trois polymères

4

0236156

à structure complexe dont les proportions varient avec l'âge et la nature de l'espèce végétale. Le complexe lignocellulosique forme une ossature tridimensionnelle où les chaînes de cellulose sont entourées et protégées par de la lignine. L'ensemble de cette structure rend ses constituants peu accessibles à une attaque enzymatique. L'attaque enzymatique du complexe lignocellulosique constitue l'étape limitante de la dégradation des composés végétaux et en particulier du processus de l'humification biologique. D'où l'intérêt de choisir parmi les microorganismes sélectionnés par le procédé décrit ci-dessus ceux auxquels le système enzymatique confère un pouvoir ligninolytique et cellulolytique important.

Or, il apparaît que les phases thermophiles II et III de compostage (comme cela sera explicité dans les exemples) présentent des caractéristiques intéressantes :

- ce sont ces phases thermophiles qui induisent la plus grande diminution de la teneur en matières organiques (en pourcentage de la teneur en matières minérales) ;

- le dosage de la lignine et de la cellulose montre que la flore microbienne présente une activité cellulolytique et ligninolytique importante ;

- elles correspondent aux phases maximales de l'activité cellulolytique au cours de l'humification.

En conséquence, le choix des microorganismes pour l'ensemencement va se porter sur ceux sélectionnés au cours des phases II et III.

En particulier, la composition microbienne selon la présente invention comporte, de préférence, des souches choisies parmi les genres Bacillus et Xanthomonas.

Ainsi, la composition microbienne selon l'invention comporte, de préférence, les souches de Bacillus subtilis 310.2 Xanthomonas 311 et Xanthomonas 320.3.

Dans le cadre de la présente invention, le support peut être utilisé, notamment, pour assurer la conservation des microorganismes et ce, non seulement pour les inoculer, mais aussi pour les transporter et surtout pour les stocker pendant les périodes plus ou moins longues.

En effet, la plupart des microorganismes utiles lors du compostage ont été isolés et cultivés dans le but d'être inoculés en vue d'activer ce compostage. Mais, un problème majeur, rencontré lors de la manipulation de microorganismes, est leur mode de transport, de conservation et d'utilisation.

Ce problème se rencontre aussi lorsqu'on veut appliquer à des graines végétales un produit microbien, ou lorsqu'on veut améliorer les qualités fertilisantes d'un engrais. De même, si l'on veut reconstituer la flore microbienne d'un sol stérile, régénerer un sol par la formation d'humus, ou encore inoculer des microorganismes fixateurs d'azote atmosphérique dans le but de régénérer un sol par la formation d'azote assimilable, on se heurte au problème de la forme d'inoculation, de transport et de stockage des microorganismes.

Pour améliorer la symbiose, les microorganismes peuvent être associés à Bacillus 321.2, Xanthomonas 310 et Xanthomonas 321, ces trois souches présentant des activités intéressantes permettant d'activer le procédé d'humification.

Cet inoculum microbien peut comporter d'autres microorganismes, notamment des bactéries fixatrices d'azote atmosphérique telles que Azotobacter, ces bactéries devant être de préférence mésophiles et thermotolérantes pour supporter la phase thermophile. Ceci est possible puisque de tels microorganismes apparaissent naturellement durant le compostage. Ces microorganismes vont permettre, en fin de compostage, d'augmenter la teneur en azote du compost.

D'autres microorganismes peuvent également être ajoutés pour la symbiose tels que Klebsiella et Sporocytophaga.

En outre, cet inoculum comporte de préférence un support microbien constitué de pulpe de raisin séchée, broyée.

C'est pourquoi la présente invention concerne également un tel inoculum microbien comportant en outre à titre de support microbien de la pulpe de raisin séchée et broyée.

L'un des buts de la présente invention est donc de proposer un matériau-support de ces microorganismes, permettant leur manipulation sous forme d'inoculums.

Actuellement, les principaux inoculums comportent de la tourbe humide ou des granulés de tourbe. On a aussi fait appel à des particules de cellulose, à un milieu de culture (comme dans le brevet des Etats Unis d'Amérique n° 3 115 404), des granulés de plâtre (brevet français n° 1 490 046) et de la lignite.

Depuis peu, on utilise divers procédés d'inclusion dans une matrice de polymère tel que des polysaccharides (brevets européens n° 0 017 565 et 0 125 073).

Il a été récemment proposé l'utilisation d'une matière organique d'origine forestière ou agricole compostée (brevet français n° 2 559 478).

Aucune de ces solutions ne parvient à résoudre simultanément tous les problèmes de l'inoculation, du transport ou du stockage des microorganismes.

C'est pourquoi la présente invention concerne un inoculum microbien utile notamment à l'accélération du compostage, à l'amélioration des sols et/ou à l'enrobage des graines, caractérisé en ce que le support microbien est constitué essentiellement par de la pulpe de raisin séchée broyée.

La pulpe de raisin séchée broyée apparaît être une solution préférable aux solutions antérieures grâce à ses nombreux avantages :

- c'est une matière organique biodégradable et non polluante ;

- elle présente des aptitudes à la libération des microorganismes et à leur adaptation dans le milieu ;

- son essaimage, que ce soit dans un sol ou un engrais, est aisé et assure une bonne pénétrabilité du milieu par les racines ;

- cette matière de très fine granulométrie et à fort pouvoir adsorbant assure convenablement la survie du microorganisme et sa protection.

La pulpe de raisin séchée broyée selon la présente invention possède une granulométrie inférieure à 0,5 mm et de préférence inférieure à 0,1 mm.

La granulométrie très fine et le fort pouvoir adsorbant de la pulpe de raisin séchée broyée permettent d'utiliser ce produit en tant que fixateur de cellules microbiennes. Ainsi, l'inoculum selon la présente invention peut comporter $10^7$ à $10^{10}$, et de préférence $10^8$ à $10^9$, cellules microbiennes par gramme d'inoculum.

De plus, il s'agit d'un produit bon marché et facilement disponible. En particulier, on peut obtenir à partir de marcs de vinification et de distillation deux formes de pulpe de raisin; une forme sèche contenant environ 10 % d'humidité, épépinée et broyée finement, c'est essentiellement à partir de cette forme sèche que l'on réalise des inoculums selon la présente invention ; la seconde forme est une forme humide contenant environ 60 à 65 % d'humidité, épépinée et broyée.

On peut éventuellement additionner cette pulpe humide à la forme sèche pour réaliser un inoculum selon la présente invention.

En outre, selon la présente invention, on peut aussi utiliser de la rafle de raisin à titre de support additionnel de l'inoculum microbien.

Quant aux microorganismes à inoculer, il s'agit de préférence de bactéries choisies en fonction de la destination de l'inoculum. Ainsi, on peut utiliser des bactéries fixatrices d'azote, du genre Rhizobium, ou des bactéries connues pour leurs propriétés humificatrices telles que Bacillus ou Xanthomonas.

En particulier, lors de compostages, il est intéressant de pouvoir utiliser des inoculums contenant les microorganismes déjà mentionnés ci-dessus, à savoir, choisis parmi Bacillus 310.2, Xanthomonas 311 et Xanthomonas 320.3, éventuellement associés à des microorganismes choisis parmi Bacillus 321.2, Xanthomonas 310 et Xanthomonas 321, en mélange éventuel avec des bactéries fixatrices d'azote telles que Azobacter, en particulier de telles bactéries thermorésistantes.

Un tel inoculum appliqué avant le début des étapes thermophiles du compostage permet de diminuer les temps de compostage.

D'autres microorganismes peuvent être utilisés dans ce but, notamment Klebsiella et Sporocytophaga.

C'est pourquoi la présente invention s'étend à l'application de ces inoculums pour le compostage et également pour l'amélioration des sols par inoculation directe du sol ou bien pour l'enfouissement avec des déchets végétaux, notamment d'origine viticole, tels que la pulpe de raisin, sèche, humide ou les rafles de raisin.

La présente invention s'étend également à l'utilisation de la pulpe de raisin séchée broyée comme milieu de culture de microorganismes, quelle que soit sa destination.

La composition chimique lui permet, en effet, d'être utilisée comme milieu d'enrichissement pour la croissance en milieu liquide ou gélosé de microorganismes.

Eventuellement, on peut lui adjoindre des éléments non présents et utiles à la croissance de microorganismes spécialistes de la dégradation des végétaux, à savoir ceux doués d'activités cellulolytiques et/ou ligniholytiques.

Il est également possible d'adjoindre à ce support des éléments utiles au développement de végétaux, tels que des oligoéléments ou des vitamines et/ou des produits de traitement phytosanitaires tels que des herbicides sélectifs.

L'inoculum microbien selon la présente invention
est préparé, de préférence, de la façon suivante :

a) on centrifuge le milieu de culture dans
lequel on a préalablement cultivé le microorganisme désiré,

b) on effectue un mélange homogène du culot de
centrifugation obtenu à l'issue de l'étape a) avec de la
pulpe séchée broyée.

Enfin, la pulpe de raisin séchée broyée et micronisée peut être utilisée dans le milieu de conservation pour
la lyophilisation de microorganismes.

En effet, pour une concentration de 25 % (poids/
volume) dans un milieu conservateur contenant des cryo-protecteurs classiques comme le sucrose, le glutamate de sodium, ou
les glycérides, la pulpe de raisin broyée et micronisée (affinement des grains par un broyage plus poussé) améliore la conservation des microorganismes lyophilisés et notamment celle
des non sporulants et permet une facile récupération de la poudre lyophilisée.

La pulpe de raisin séchée broyée possède donc un
rôle de support matériel absorbant et le rôle de charge du lyophilisat comme la silice (brevet français n° 1 584 757) ou la
cellulose (brevet français n° 1 426 226). Ce matériau présente
aussi l'intérêt de la protection du microorganisme lors de la
réhydratation du lyophilisat.

Dans le procédé de compostage selon la présente
invention, l'inoculation doit être réalisée au début de la
phase thermophile au cours du processus. Elle peut être effectuée à raison d'une concentration finale dans la matière à humifier de $10^6$ cellules/g.

Si l'on utilise comme support de l'inoculum la
pulpe de raisin séchée broyée, l'évolution de la microflore
au cours du compostage aéré montre une grande capacité de ces
microorganismes à s'adapter et à se multiplier dans la matière
végétale.

En effet, le taux de croissance de la microflore au cours de la phase thermophile est augmenté de 36 %.

Il faut noter que l'inoculation exerce un effet sur la phase de maturation du compost. Bien que le taux de décroissance soit le même, la quantité de cellules est largement supérieure à celle observée lors d'une humification aérée mais non activée. En effet, la biomasse est augmentée de 30 % ; l'effet de l'inoculation est donc durable.

La propagation des microorganismes inoculés est mise en évidence par une augmentation de l'activité cellulosique au cours de l'humification. Cette activité présente deux maxima qui sont augmentés respectivement de 35 % et de 64 %.

L'évolution du taux du rapport carbone/azote (C/N) reflète la dégradation de la matière organique. La mesure de ce taux montre que l'activation par inoculation des souches bactériennes sélectionnées augmente de façon très significative la biodégradation. Ce taux est abaissé à 11,1, au lieu de 12,6, pour un compostage sans inoculation.

La mise en oeuvre des différents moyens mentionnés précédemment, en particulier l'oxygénation et l'utilisation d'inoculums microbiens particuliers, permet de réduire de façon très significative les durées de compostage tout en obtenant un produit de qualité identique à celui obtenu par compostage traditionnel.

Les exemples ci-après sont destinés à illustrer d'autres caractéristiques et avantages du procédé de compostage selon l'invention, ainsi que le mode de fabrication et l'analyse chimique de l'inoculum microbien comportant à titre de support de la pulpe de raisin séchée broyée.

EXEMPLE 1 :

Analyse des différentes phases de compostage de la pulpe sèche et de la pulpe humide

Rôle de l'aération

On a transposé à l'échelle du laboratoire le processus de compostage utilisé par l'industriel par l'utilisation d'un réacteur de 100 l équipé d'un système d'oxygénation contrôlé.

Ainsi, à partir de mini-compost, on a pu mesurer les paramètres classiques de compostage (température, pH, humidité, taux de C/N, oxygénation) auxquels on a ajouté la mesure par bioluminescence utilisant le système luciférine-luciférase de la concentration en ATP qui reflète la biomasse active.

L'utilisation d'un mini-compost permet à 20 cm l'observation caractéristique d'un compostage classique : temps de latence, montée brutale en température jusqu'à 60°C, stabilisation en température, puis descente lente jusqu'à maturation du compost.

La durée du compostage est de 9 jours et permet ainsi de réaliser plusieurs expériences rapprochées.

Le pH varie de 7 (début du compostage) à 8,5 (3 ou 4 jours après) et se stabilise en fin de fermentation.

Le taux d'humidité baisse légèrement indiquant que le départ d'eau durant la phase thermophile est en large partie (mais non totalement) compensé par la production d'eau au cours de la dégradation aérobie des macromolécules organiques.

Le rapport C/N s'abaisse de 17 à 13. Il diminue plus rapidement dans la première moitié de la phase thermophile que dans la deuxième moitié. La diminution de ce rapport met en évidence la dégradation des macromolécules aboutissant au dégagement de $CO_2$.

Les phases de biomasse activée lors du compostage ont pu être déterminées par la méthode de détermination de la concentration d'Adénosine triphosphate (ATP).

Les méthodes classiques d'évaluation de la biomasse comme la mesure de la concentration en protéines et l'activité de diverses enzymes comme la phosphatase alcaline sont faussées par la présence de formes mortes ou inactives de microorganismes.

Une méthode récente est le dosage de l'ATP afin d'estimer la biomasse totale active. Cette méthode est basée sur le fait que les cellules microbiennes contiennent une quantité relativement constante d'ATP et que cette molécule peut être dosée par la réaction luciférine-luciférase. La mesure quantitative d'ATP à des niveaux mêmes faibles, par bioluminescence, est relativement simple, rapide et sensible.

Cependant, l'extraction de l'ATP dans des échantillons naturels présente un problème certain. La méthode présentée ici est le résultat de l'essai de différentes méthodes et d'une mise au point qui a permis d'obtenir une détermination fiable ($\pm$ 6 % d'erreur relative).

La méthode d'extraction est réalisée à partir de 1 g d'échantillon en utilisant l'ultrasonication dans un milieu extractant qui est le diméthylsulfoxide (DMSO).

L'extrait est stocké dans un tampon conservateur Gly/Mg EDTA, pH = 7,5, après congélation rapide.

La mesure est effectuée de façon simple avec un kit commercial de réactifs pour détermination d'ATP et un bioluminomètre.

La détermination de la concentration en ATP a permis de mettre en évidence 5 phases principales de développement de microorganismes (tableau I) de courte durée (de quelques heures à un jour), constantes, se retrouvant aux profondeurs de 10 à 20 cm.

Les phases I et IV sont plus intenses et leur importance varie principalement en fonction du lot de pulpe utilisé.

14

0236156

## TABLEAU I
## PRINCIPALES PHASES DE COMPOSTAGE DE LA
## PULPE SECHE PAR LE FUMIER DE MOUTON (2 : 1)

### (Paramètres déterminés aux pics de biomasse)

| Phase | Temps (jours) | Température (°C) | pH | Microflore |
|-------|---------------|------------------|-----|-------------|
| I | 1,2 | 45 | 7,5 | mésophile |
| II | 2,5 | 58 | 8 | thermophile |
| III | 4 | 60 | 8,5 | thermophile |
| IV | 6 | 54 | 8,9 | thermophile |
| V | 8 à 9 (biphasique) | 25-30 | 8,9 | mésophile |

### EXEMPLE 2
### Effet de l'aération

L'aération par insufflation d'air toutes les 24 heures permettant un renouvellement total à l'intérieur du compost, fait apparaître plusieurs différences par rapport aux conditions non aérées.

L'aération accélère donc le processus et conduit à un gain de temps pour la fermentation de 25 à 30 %.

La température augmente plus rapidement et plafonne à des valeurs plus élevées (62 °C au lieu de 55°C).

Le rapport C/N diminue plus rapidement mais atteint les mêmes valeurs en fin de fermentation.

Le pH augmente plus lentement mais atteint des valeurs comparables (environ 8,5).

L'aération double la quantité de biomasse pour toutes les phases sauf la phase V (tableau II).

**TABLEAU II**

| Phase | Vitesse de consommation $O_2$ ($\% O_2$/min) | Quantité de biomasse (nmole d'ATP/g d'échantillon) | |
|-------|-----------------------------|-----------|-------|
| | | non aéré | aéré |
| I | 0,02 | 38 | 110 |
| II | 0,10 | 25 | 110 |
| III | 0,30 | 50 | 140 |
| IV | 0,52 | 100 | 200 |
| V | 0,22 à 0,10 | 100 à 75 | 60 à 30 |

La phase IV correspond à une demande très importante en oxygène (0,5 % d'$O_2$/minute) qui annonce la fin du processus thermophile de compostage et la maturation du milieu.

La phase V ne dépend pas de l'oxygénation et ne doit pas correspondre à une étape de dégradation des macromolécules organiques. Elle se situe à la fin de la fermentation et présente une microflore importante et variée. La consommation en oxygène diminue mais ne devient pas nulle. Elle est même plus importante que dans les trois premières phases.

L'oxygène consommé dans cette dernière phase ne semble pas être utilisé pour le développement de la biomasse, mais plutôt dans des phénomènes d'oxydation permettant par exemple la synthèse des acides humiques.

Dans des conditions d'aération, l'humification d'une matière organique préalablement stockée dans des conditions d'anaérobiose est fortement diminuée. L'absence de deux des cinq phases prévues met en évidence l'importance de la présence de certains microorganismes aérobies pour le bon fonctionnement du processus.

En compostage industriel, l'aération toutes les 24 heures par insufflation ou par mélange permet un gain de temps de 30 à 50 %.

A partir de ces deux exemples, on a pu montrer que le compostage de la pulpe de raisin est essentiellement un processus aérobie et qu'il convient de bien maîtriser l'oxygénation pour une bonne biotransformation de ces déchets comme cela est proposé dans le procédé selon l'invention.

Notamment dans les deux phases finales, l'apport d'oxygène semble être particulièrement important pour la formation des acides humiques. Il apparaît que l'insufflation d'air est plus indiquée dans la première phase mésophile, alors que le mélange par retournement est plus efficace dans les phases ultérieures.

Il a également été mis en évidence que le taux d'humidité élevé de la pulpe humide n'est pas le seul facteur responsable de sa mauvaise dégradabilité. La présence de certaines souches bactériennes semble être indispensable au bon déroulement du processus. On a ainsi pu montrer que le compostage de la pulpe sèche faisait apparaître cinq phases biotiques principales dont deux sont absentes (ou faibles) dans le compostage de la pulpe humide.

Ce travail original sur les phases biotiques met en relief l'intérêt de l'utilisation d'un inoculum microbien dans la mise en oeuvre du procédé selon l'invention.

Les exemples suivants ont pour but de permettre de sélectionner des souches microbiennes en fonction de leurs propriétés enzymatiques pour les utiliser à titre d'inoculum dans le procédé selon l'invention.

EXEMPLE 3

## Isolement des microorganismes à partir d'échantillons présélectionnés par phase

Des échantillons de mini-composts de pulpe sèche ont été prélevés juste avant l'optimum des cinq phases et ont servi à l'ensemencement des premiers milieux de transfert. Des transferts successifs ont ensuite été réalisés avant l'isolement des souches (7 transferts).

Cette technique qui permet de sélectionner les microorganismes dominants dégradant la pulpe de raisin a été mise au point au laboratoire. Les cultures successives ont été réalisées dans les conditions de pH et de température dans lesquelles les échantillons ont été prélevés.

L'isolement montre que la sélection par phase et par transferts successifs permet l'isolement de microorganismes dominants représentés exclusivement par des bactéries et des actinomycètes. Cet isolement ne met pas en évidence de champignons dominants, ce qui pourrait être particulièrement le cas avec des composts démarrant à la neutralité pour finir à un pH relativement alcalin (pH 8,5 à 8,9).

Par contre, il est caractéristique de la dégradation des résidus végétaux. En effet, au cours de la première phase, on note la présence d'une microflore dominante et variée due à la mésophilie et à la richesse initiale en sucres libres dans la pulpe de raisin. Les phases thermophiles possèdent au contraire une microflore beaucoup moins variée mais plus spécifique. La présence d'Actinomycètes et à un degré moindre d'entérobactéries caractérise la maturation du compost.

Les parois cellulaires des végétaux sont essentiellement constituées de cellulose, d'hémicelluloses et de lignine. Il s'agit de trois polymères à structure complexe dont les proportions varient avec l'âge et la nature de
l'espèce végétale. Le complexe lignocellulosique forme une
ossature tridimensionnelle où les chaînes de cellulose
sont entourées et protégées par de la lignine. L'ensemble
de cette structure rend ses constituants peu accessibles
à une attaque enzymatique. L'attaque enzymatique du complexe
lignocellulosique constitue l'étape limitante de la dégradation des composés végétaux et en particulier du processus
de l'humification biologique. Ce problème reste résolu chez
les microorganismes ligninolytiques et cellulolytiques grâce
à un système enzymatique complexe.

Les activités cellulasique et ligninolytique ont
été recherchées et mesurées pour toutes les souches dominantes
précédemment sélectionnées.

La détermination de l'activité cellulasique a
été déterminée tout d'abord par le test de la cellulose-azure
(Poincelot, 1972) et la mesure sur filtre de papier par la
méthode de Mandels (1976).

La mesure de l'activité ligninolytique a été
déterminée par la méthode de Janshekar (1982) avec de la
lignine extraite de la pulpe de raisin.

Ce sont les phases II et III qui laissent
apparaître que des souches cellulolytiques et/ou ligninolytiques

L'ensemble des souches sélectionnées au cours de
ces deux phases sont rassemblées dans le tableau III.

# TABLEAU III

## SELECTION DE MICROORGANISMES DOMINANTS
## LORS DE L'HUMIFICATION BIOLOGIQUE DE LA PULPE DE RAISIN

|  |  |  |  | Cellulolytique | Ligninolytique |
|---|---|---|---|---|---|
| II | 50°C<br>pH = 8,5 | 310 | Xanthomonas | ++ | O |
|  |  | 310.2 | Bacillus subtilis | +++ | ++ |
|  |  | 311 | Xanthomonas | O | +++ |
|  |  | 313 | genre indéterminé | O | ++ |
| III | 50°C<br>pH = 8,5 | 320.3 | Xanthomonas | O | +++ |
|  |  | 321 | Xanthomonas | ++ | + |
|  |  | 321.2 | Bacillus | O | ++ |
|  |  | 323 | Bacillus | O | ++ |

19

0236156

D'autre part, ces deux phases ont des caractères intéressants :

- ce sont les phases thermophiles qui induisent la plus grande diminution de la teneur en matières organiques (en pourcentage de la teneur en matières minérales) ;

- le dosage de la lignine et de la cellulose montre qu'elles possèdent une activité cellulolytique et ligninolytique importante ;

- elles correspondent aux phases maximales de l'activité cellulolytique au cours de l'humification.

En conséquence, le choix des microorganismes pour l'ensemencement va se porter sur ceux existant dans les phases II et III.

La détermination taxonomique a montré qu'il s'agissait :

Phase II    : Bacillus subtilis 310.2

Xanthomonas 311

Xanthomonas 310

Phase III   : Xanthomonas 320.3

Xanthomonas 321

Bacillus subtilis 321.2.

EXEMPLE 4

Détermination des propriétés biochimiques

des souches sélectionnées

La mesure des activités cellulolytique et ligninolytique a montré que trois de ces souches possédaient des activités enzymatiques remarquables.

C'est pourquoi on a comparé avec des activités enzymatiques de souches bactériennes reconnues pour leurs performances et qui ont servi de référence.

## Activité cellulolytique

| | Activité enzymatique dans l'essai (mg Glc libéré/h) | Activité spécifique pour $10^7$ cellules (mg Glc libéré/h) |
|---|---|---|
| - Bacillus 310.2 | 1,62 | 0,097 |
| - Sporocytophaga myxococcoïdes (collection DSM GBR) | 1,04 | 0,086 |
| - Cytophaga 1761 hutchinsonii (collection DSM,GBR) | 0,60 | 0,043 |

## Activité ligninolytique

| | Dégradation de la lignine après 10 jours de culture (%) |
|---|---|
| - Xanthomonas 311 | 68 |
| - Xanthomonas 320.3 | 66 |
| - Rhodococcus erythropolis (collection DSM, GBR) | 30 |

EXEMPLE 5

Fabrication d'un inoculum

Après culture du microorganisme à inoculer dans un erlenmeyer, on centrifuge le milieu de culture. On effectue ensuite un mélange homogène du culot de centrifugation avec de la pulpe séchée broyée, à raison de $5.10^{11}$ cellules microbiennes pour 10 g de pulpe séchée broyée.

Le produit $M_1$ obtenu est alors mélangé intimement avec 500 g de support constitué essentiellement de pulpe séchée broyée.

L'inoculum ainsi préparé contient $10^9$ cellules par gramme d'inoculum.

EXEMPLE 6

Analyse chimique de l'inoculum

- humidité : 10 %
- pH : 6,5

| | | |
|---|---|---|
| - matières organiques totales, | 89,7 % du poids sec | |
| - matières minérales, | 10,3 % | " " |
| - cellulose, | 13,9 % | " " |
| - hémicellulose, | 14,1 % | " " |
| - lignines | 35,1 % | " " |
| - matières protéiques, | 17,4 % du poids sec | |
| - carbone, | 49,6 % | " " |
| - azote total, | 2,6 % | " " |
| - carbone/azote, | 19,0 | |
| - phosphore total (en $P_2O_5$), | 0,59% | " " |
| - potassium total (en $H_2O$), | 2,3 % | " " |
| Dénombrement des cellules microbiennes viables, | $10^6$ cellules/g. | |

EXEMPLE 7

Essais

Il est bien évident que la survie des microorganismes fixés dépend de leur type et de leur genre.

Les études de survie d'un Bacillus subtilis fixé montrent une survie quasi totale pour un temps inférieur à un mois et un stockage dans un endroit sec et frais. Pour un temps supérieur (jusqu'à deux mois de stockage) un inoculum de $6.10^8$ cellules/g passe à une concentration de $4.10^8$ cellules/g.

Pour une fabrication intensive et un stockage long, il apparaît préférable de stocker l'inoculum à une concentration plus élevée et dans une chambre froide (+ 4°C).

Un mélange ultérieur avec de la pulpe séchée broyée permettra de le diluer.

EXEMPLE 8

Lors d'un compostage de pulpe de raisin l'inoculation d'un inoculum selon l'invention contenant :

- Bacillus subtilis 310.2
- Xanthomonas 311
- Xanthomonas 320.3

avant la phase thermophile permet de diminuer de 1/3 le temps de compostage.

0236156

Les souches suivantes ont été déposées à la Collection Nationale de Cultures de Microorganismes de l'INSTITUT PASTEUR, 28 rue du Docteur-Roux, 75724 Paris Cédex 15, le 17 janvier 1986 :

- Bacillus subtilis 310.2, sous le n° I-504
- Xanthomonas 311 primitivement identifié comme étant Pseudomonas 311, sous le n° I-505
- Xanthomonas 320.3 primitivement identifié comme étant Flavobacterium multivorum 320.3, sous le n° I-506.

## REFERENCES

JANSHEKAR H. et FIECHTER A. On the Bacterial Degradation of lignin. Eur. J. Appl. Microbiol. Biotechnol. 14, 47-50 (1982)

MANDELS M. ANDREOTTI R. et ROCHE C. Measurement of Saccharifying Cellulase. Biotechnol. and Bioeng. Symp. 6, 21-33 (1976)

POINCELOT R.P. et DAY P.R. Simple Dye Release Assay for Determining Cellulolytic Activity of fungi. Appl. Microbiol. 4, 875-879 (1972)

0236156

## REVENDICATIONS

1) Procédé de compostage de matières organiques d'origine végétale, caractérisé en ce que :

a) on prépare un tas de matières à composter contenant des matières organiques d'origine végétale et éventuellement des matières organiques d'origine animale,

b) on insuffle de l'oxygène dans le tas de matières à composter,

c) à l'apparition de la phase thermophile du compostage, on procède à l'aération mécanique régulière du tas,

d) l'aération mécanique est arrêtée avant la fin de la phase thermophile.

2) Procédé selon la revendication 1, caractérisé en ce que l'oxygène est insufflé sans que le tas soit demembré.

3) Procédé selon l'une des revendications 1 et 2, caractérisé en ce que l'oxygène est insufflé par l'intermédiaire de cannes perforées qui sont enfoncées dans le tas.

4) Procédé selon l'une des revendications 1 à 3, caractérisé en ce qu'on ajoute au tas un inoculum microbien contenant des bactéries à activité cellulolytique et ligninolytique.

5) Procédé selon la revendication 4, caractérisé en ce que l'inoculum est ajouté au début de la phase thermophile.

6) Procédé selon l'une des revendications 4 et 5, caractérisé en ce que l'inoculum bactérien contient des bactéries choisies parmi : Bacillus et Xanthomonas.

7) Procédé selon la revendication 6, caractérisé en ce que l'inoculum bactérien contient des bactéries choisies parmi : Bacillus 310.2, Xanthomonas 311 et Xanthomonas 320.3.

8) **Procédé selon la revendication 7, caractérisé
en ce que l'inoculum** contient, en outre, au moins
**une bactérie choisie parmi :** Bacillus 321.2, Xanthomonas 310
et Xanthomonas 321.

9) **Procédé selon l'une des revendications** 4 à 8,
caractérisé en ce que l'inoculum contient, en outre, des
bactéries fixatrices d'azote gazeux.

10) **Procédé selon la revendication 9, caractérisé
en ce que les bactéries fixatrices d'azote sont choisies dans
le genre Azotobacter, mésophile et thermorésistant.**

11) **Procédé selon l'une des revendications 1 à 10,
caractérisé en ce que les matières organiques d'origine
végétale sont constituées de pulpe de raisin.**

12) **Procédé selon l'une des revendications 1 à 11,
caractérisé en ce que les matières organiques contiennent, en
outre, du fumier de mouton.**

13) **Procédé selon l'une des revendications 4 à 12,
caractérisé en ce que l'inoculum** microbien est constitué de microorganismes sur un support de pulpe de raisin séchée et broyée.

14) **Inoculum microbien utile notamment à
l'accélération du compostage, à l'amélioration des sols
et/ou à l'enrobage des graines, caractérisé en ce que le
support microbien est constitué essentiellement par de
la pulpe de raisin séchée, broyée.**

15) **Inoculum selon la revendication 14,
caractérisé en ce que la pulpe de raisin séchée broyée
possède une granulométrie inférieure à 0,5 mm.**

16) **Inoculum selon l'une des revendications**
14 et 15, caractérisé en ce que la pulpe de raisin séchée
broyée possède une granulométrie inférieure à 0,1 mm.

17) **Inoculum selon l'une des revendications**
14 à 16, caractérisé en ce qu'il comporte de $10^7$ à $10^{10}$
cellules microbiennes par gramme d'inoculum.

18) **Inoculum selon l'une des revendications**
14 à 17, caractérisé en ce qu'il comporte de $10^8$ à $10^9$
cellules microbiennes par gramme d'inoculum.

19 ) Inoculum selon l'une des revendications 14 à 18, caractérisé en ce que la pulpe de raisin séchée broyée est obtenue à partir de marcs de vinification et de distillation.

20 ) Inoculum selon l'une des revendications 14 à 19, caractérisé en ce que le support microbien comporte en outre de la pulpe de raisin humide broyée et/ou des rafles.

21 ) Inoculum selon l'une des revendications 14 à 20, caractérisé en ce que le support microbien comporte en outre les éléments nutritifs spécifiques à la croissance de microorganismes et notamment ceux doués d'activités cellulolytiques et/ou ligninolytiques.

22 ) Inoculum selon l'une des revendications 14 à 21, caractérisé en ce que le support microbien comporte, en outre, des éléments nutritifs ou utiles au développement des végétaux et/ou des produits de traitement phytosanitaires.

23) Inoculum selon l'une des revendications 14 à 22, caractérisé en ce que les microorganismes sont des bactéries.

24) Inoculum selon l'une des revendications 14 à 23 caractérisé en ce qu'il comporte au moins une souche choisie parmi les genres Bacillus et Xanthomonas.

25) Inoculum selon la revendication 24, caractérisé en ce qu'il comporte au moins une souche choisie parmi :

- Bacillus 310.2
- Xanthomonas 311
- Xanthomonas 320.3.

26) Inoculum selon l'une des revendications 24 et 25, caractérisé en ce qu'il comporte au moins une bactérie fixatrice d'azote atmosphérique.

27) Inoculum selon l'une des revendications 24 à 26 caractérisé en ce qu'il comporte en outre au moins un microorganisme choisi parmi Klebsiella et Sporocytophaga.

28) Application de l'inoculum selon l'une des revendications 14 à 27 pour l'ensemencement de composts.

29) Application de l'inoculum selon l'une des revendications 14 à 27 pour l'ensemencement de sols.

30) Utilisation de la pulpe de raisin séchée, broyée dans un milieu de conservation pour la lyophilisation des micro-organismes.